# EUROPEAN PATENT APPLICATION

(11) **EP 4 799 672 A1**
(43) Date of publication of application: **02.09.2026**
(21) Application number: 24881463.4
(22) Date of filing: 14.10.2024
(51) Int. Cl.: A61M 25/10

(54) **BALLOON SHEATH CATHETER**

(30) Priority: 27.10.2023 CN 202311418329
(71) Applicant: Shenzhen Wecan Medical Technology Co., Ltd., Shenzhen, Guangdong 518063 (CN)
(72) Inventor: ZHANG, Wei, Shenzhen, Guangdong 518063 (CN); CHEN, Zhong, Shenzhen, Guangdong 518063 (CN); WU, Liping, Shenzhen, Guangdong 518063 (CN); ZHANG, Xiong, Shenzhen, Guangdong 518063 (CN); LI, Zhen, Shenzhen, Guangdong 518063 (CN); HAO, Chenxiang, Shenzhen, Guangdong 518063 (CN)
(74) Representative: Michalski Hüttermann & Partner mbB
(86) International application number: PCT/CN2024/124746
(87) International publication number: WO 2025/087097

(57) **Abstract**

A balloon sheath catheter (100), including: a sheath catheter main body (110), which is provided with a main lumen (111), an inflation lumen (112) and a perfusion lumen (113) that are not in communication with each other, where a distal end of the main lumen (111) penetrates through a distal end of the sheath catheter main body (110), at least one first side hole (114) and at least one second side hole (115) are formed on a side wall of the sheath catheter main body (110), and the first side hole (114) is in communication with the perfusion lumen (113) and an external space of the sheath catheter main body (110); and a balloon (120), which is arranged near a distal end of the balloon sheath catheter (100), where the second side hole (115) is in communication with the balloon (120) and the inflation lumen (112), and the first side hole (114) is formed on a proximal side of the balloon (120). The balloon (120) of the balloon sheath catheter (100) is used for occluding a blood vessel, and the first side hole (114) formed on the proximal side of the balloon (120) and the perfusion lumen (113) in communication with the first side hole (114) are used for perfusion of a contrast agent. The contrast agent can show whether the blood vessel is completely occluded by the balloon (120), without the need to insert an additional angiographic catheter from another part of the blood vessel.

## Description

### Technical Field

The present invention relates to the technical field of medical equipment, in particular to a balloon sheath catheter.

### Background Art

From the 1970s to today, vascular interventional therapy technology has developed vigorously and rapidly. Sheath catheters play a significant role in establishing surgical instrument pathways. Since many vascular interventional surgeries require the occlusion of blood flow, balloon sheath catheters have been developed.

As for the current balloon sheath catheters, their structures mostly involve establishing an inflation lumen on a wall of the sheath catheter to inflate a balloon. Moreover, the balloon sheath catheters are used primarily to occlude a blood vessel and stop the blood flow. To monitor the blood pressure at the posterior end of the balloon or determine whether the balloon is completely occluded, an additional angiographic catheter needs to be inserted from another blood vessel.

In conclusion, in order to optimize the existing balloon sheath catheters, it is particularly important to develop an angiographic balloon sheath catheter.

### Summary of the Invention

Based on this, it is necessary to provide an angiographic balloon sheath catheter.

The present invention provides a balloon sheath catheter, including:
a sheath catheter main body, which is provided with a main lumen, an inflation lumen and a perfusion lumen that are not in communication with each other, where a distal end of the main lumen penetrates through a distal end of the sheath catheter main body, at least one first side hole and at least one second side hole are formed on a side wall of the sheath catheter main body, and the first side hole is in communication with the perfusion lumen and an external space of the sheath catheter main body; and
a balloon, which is arranged near a distal end of the balloon sheath catheter, where the second side hole is in communication with the balloon and the inflation lumen, and the first side hole is formed on a proximal side of the balloon.

In one embodiment, the aperture of the first side hole gradually decreases from inside to outside along a radial direction of the sheath catheter main body.

In one embodiment, the sheath catheter main body includes an inner layer, a support layer and an outer layer that are arranged radially from inside to outside;
the support layer includes at least one support rod, where the support rod helically extends along an axial direction of the sheath catheter main body, or the support rod is woven to form the support layer.

In one embodiment, the inflation lumen and/or the perfusion lumen are embedded into the outer layer.

In one embodiment, the support rod is a hollow tube, and the lumen of the support rod forms at least one of the perfusion lumen or the inflation lumen.

In one embodiment, a valve body is provided in the perfusion lumen, and when the fluid pressure in the perfusion lumen is greater than the preset pressure of the valve body, the valve body opens to allow fluid to flow from the distal end of the sheath catheter main body to a proximal end of the sheath catheter main body.

In one embodiment, a plurality of valve bodies are provided in the perfusion lumen, and the preset pressures of the plurality of valve bodies increase gradually along a direction from the distal end to the proximal end of the sheath catheter main body.

In one embodiment, the valve body is arranged near a proximal end of the perfusion lumen.

In one embodiment, the outer layer includes at least one transparent section, and the projection of the valve body along the radial direction of the sheath catheter main body falls on the transparent section to facilitate the observation of the valve body through the transparent section.

In one embodiment, the balloon sheath catheter further includes a first angiographic joint and a second angiographic joint; the first angiographic joint is arranged on a proximal side of the sheath catheter main body and is in communication with the main lumen; and the second angiographic joint is arranged on the proximal side of the sheath catheter main body and is in communication with the perfusion lumen.

The balloon of the balloon sheath catheter in the present invention is used for occluding a blood vessel, and the first side hole formed on the proximal side of the balloon and the perfusion lumen in communication with the first side hole are used for perfusion of a contrast agent. The contrast agent can show whether the blood vessel is completely occluded by the balloon, without the need to insert an additional angiographic catheter from another part of the blood vessel. Moreover, the perfusion lumen can also be used for perfusion of medicinal liquid for local treatment of the blood vessel.

### Brief Description of the Drawings

FIG. 1 is a partial axial sectional view of a balloon sheath catheter in embodiment 1 of the present invention;
FIG. 2 is a schematic diagram showing the structural connection of a balloon sheath catheter in embodiment 1 of the present invention when used in a carotid reflux system;
FIG. 3 is a schematic structural diagram of a balloon sheath catheter in embodiment 1 of the present invention;
FIG. 4 is an enlarged view of part A in FIG. 1;
FIG. 5 is a partial schematic diagram of first side holes in another embodiment of the present invention;
FIG. 6 is a radial sectional view of a balloon sheath catheter in embodiment 1 of the present invention;
FIG. 7 is a schematic structural diagram of a valve body in embodiment 1 of the present invention;
FIG. 8 is a schematic structural diagram of a valve body in a closed state in another embodiment of the present invention;
FIG. 9 is a schematic structural diagram of a valve body in an open state in another embodiment of the present invention;
FIG. 10 is a partial axial sectional view of a balloon sheath catheter in embodiment 2 of the present invention;
FIG. 11 is a schematic structural diagram of a support layer in embodiment 3 of the present invention; and
FIG. 12 is a partial axial sectional view of a balloon sheath catheter in embodiment 4 of the present invention.

### Detailed Description of the Invention

To make the purpose, the technical solution and the advantages of the present invention more clear, the present invention will be further described below in detail in combination with the drawings and the embodiments. It should be understood that the specific embodiments described herein are only used for explaining the present invention, not used for limiting the present invention.

Unless otherwise defined, all technical and scientific terms used herein have the same meanings as those generally understood by those skilled in the art in the present invention. The terms used in the description of the present invention herein are intended to merely describe the specific embodiments, not to limit the present invention. The term "and/or" used herein includes any and all of combinations of one or more related listed items.

To describe the structure of the present application more clearly, the terms "proximal end" and "distal end" are defined here as commonly used terms in the field of interventional medical treatment. Specifically, the "distal end" indicates an end away from operators during surgical operation, the "proximal end" indicates an end near the operators during the surgical operation, the "axial direction" indicates a length direction, and the "radial direction" indicates a direction perpendicular to the "axial direction".

### Embodiment 1

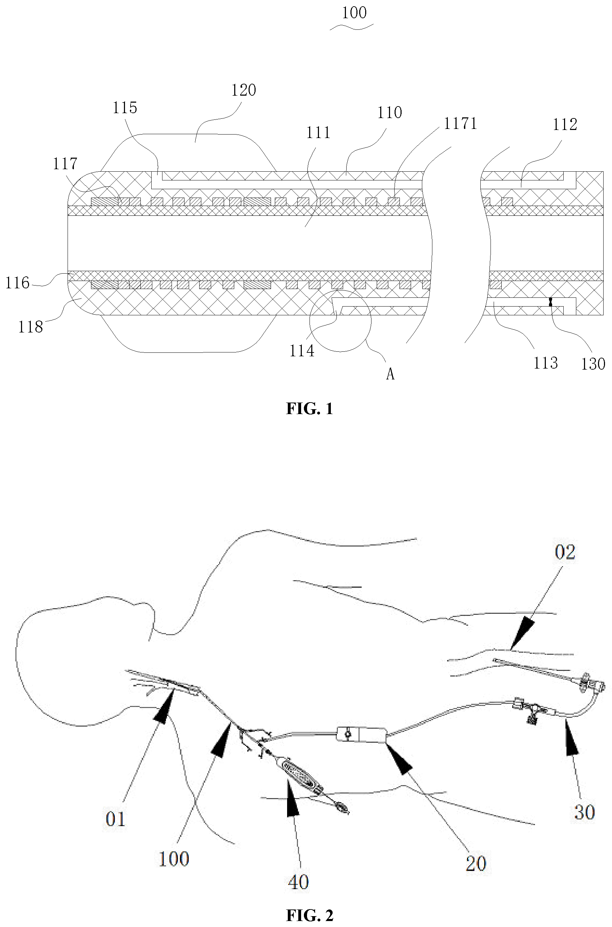

As shown in FIG. 1, this embodiment provides a balloon sheath catheter 100, including: a sheath catheter main body 110, which is provided with a main lumen 111, an inflation lumen 112 and a perfusion lumen 113 that are not in communication with each other, where a distal end of the main lumen 111 penetrates through a distal end of the sheath catheter main body 110, at least one first side hole 114 and at least one second side hole 115 are formed on a side wall of the sheath catheter main body 110, and the first side hole 114 is in communication with the perfusion lumen 113 and an external space of the sheath catheter main body 110; and
a balloon 120, which is arranged near a distal end of the balloon sheath catheter 100, where the second side hole 115 is in communication with the balloon 120 and the inflation lumen 112, and the first side hole 114 is formed on a proximal side of the balloon 120.

The balloon 120 of the balloon sheath catheter 100 in the present invention is used for occluding a blood vessel, and the first side hole 114 formed on the proximal side of the balloon 120 and the perfusion lumen 113 in communication with the first side hole 114 are used for perfusion of a contrast agent. The contrast agent can show whether the blood vessel is completely occluded by the balloon 120, without the need to insert an additional angiographic catheter from another part of the blood vessel. Moreover, the perfusion lumen 113 can also be used for perfusion of other liquid (such as normal saline and medicinal liquid) for local treatment of the blood vessel.

This embodiment takes a carotid interventional surgery as an example for illustration. As shown in FIG. 2, a carotid reflux system for the carotid interventional surgery includes the balloon sheath catheter 100, a diversion device 20, a venous sheath catheter 30, and a stent system 40. The balloon sheath catheter 100, the diversion device 20, and the venous sheath catheter 30 are connected successively, the distal end of the balloon sheath catheter 100 is inserted into a common carotid artery (a left common carotid artery 01 is illustrated below) that requires treatment, and the venous sheath catheter is inserted into a vein (a femoral vein 02 is illustrated below) of a patient. Under normal conditions, arterial blood flow starts from an aortic arch, flows to the left common carotid artery and a right common carotid artery respectively, and then flows into an intracranial cavity, and the blood in the left common carotid artery and the right common carotid artery communicates via the intracranial circle of willis. When the balloon sheath catheter 100 achieves occlusion for the left common carotid artery 01, insufficient blood supply occurs in the left common carotid artery 01. Under the effect of a pressure difference between arterial blood and venous blood, the arterial blood from the right common carotid artery flows reversely into the left common carotid artery 01 and is reinfused to a human body through the balloon sheath catheter 100, the diversion device 20, and the venous sheath catheter 30. In the process that the stent system 40 enters the left common carotid artery 01 along the balloon sheath catheter 100 and releases a stent, thrombi in the blood flow enter the diversion device 20 along with the blood flow and are filtered, which can prevent the thrombi from entering cerebral blood vessels.

In this embodiment, the balloon sheath catheter 100 further includes a first angiographic joint 130 and a second angiographic joint 140; the first angiographic joint 130 is arranged on a proximal side of the sheath catheter main body 110 and is in communication with the main lumen 111; and the second angiographic joint 140 is arranged on the proximal side of the sheath catheter main body 110 and is in communication with the perfusion lumen 113. In the process of the surgery, after the balloon sheath catheter 100 is inserted into the left common carotid artery 01, a contrast solution is first injected into the main lumen 111 through the first angiographic joint 130, and the contrast solution diffuses along the blood flow direction from the distal end of the sheath catheter main body 110 into the left common carotid artery 01. The lesion condition within the left common carotid artery 01 can be determined through DSA images, and the distal end position of the balloon sheath catheter 100 can be determined by observing the diffusion position of the contrast solution, thereby adjusting the balloon sheath catheter 100 to an appropriate position. Subsequently, an inflation medium (such as normal saline) is injected through the inflation lumen 112 to inflate the balloon 120. After inflation is completed, the balloon 120 achieves occlusion for the left common carotid artery 01. Under the effect of the pressure difference between the artery and the vein, the blood flow located at a distal end of the balloon 120 flows reversely into the main lumen 111 of the sheath catheter main body 110, and flows back into the femoral vein 02 of the patient through the main lumen 111 and the venous sheath catheter 30 in communication with the main lumen 111. Subsequently, the contrast solution is injected into the perfusion lumen through the second angiographic joint 140. Whether the contrast solution flows from the proximal side of the balloon 120 to the distal side of the balloon 120 can be determined through DSA images. If the contrast solution does not flow from the proximal side of the balloon 120 to the distal side of the balloon 120, the balloon 120 completes the occlusion for the blood vessel to ensure that the thrombi dislodged from the carotid artery wall in the process of the surgery do not enter the cerebral blood vessels along the carotid artery.

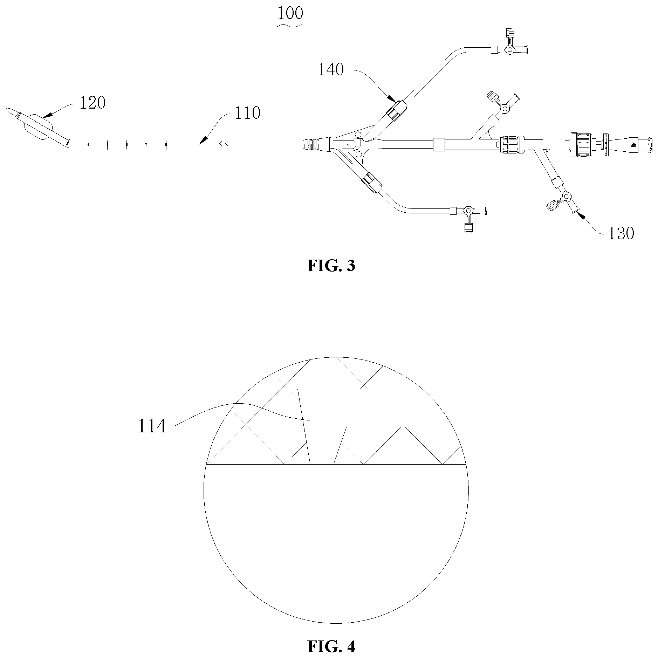

In this embodiment, as shown in FIG. 1 and FIG. 4, the aperture of the first side hole 114 gradually decreases from inside to outside along a radial direction of the sheath catheter main body 110. Since the flow rate of the liquid increases with the decrease of the flow area under the same pressure, when a contrast solution or other medicinal liquid enters the blood vessel through the first side hole 114, the flow rate of the contrast solution or other medicinal liquid increases, and the contrast solution or other medicinal liquid flows out of the first side hole 114 in a jet-like manner, and rapidly diffuses within the blood vessel, which is conducive to reducing the observation time required for imaging.

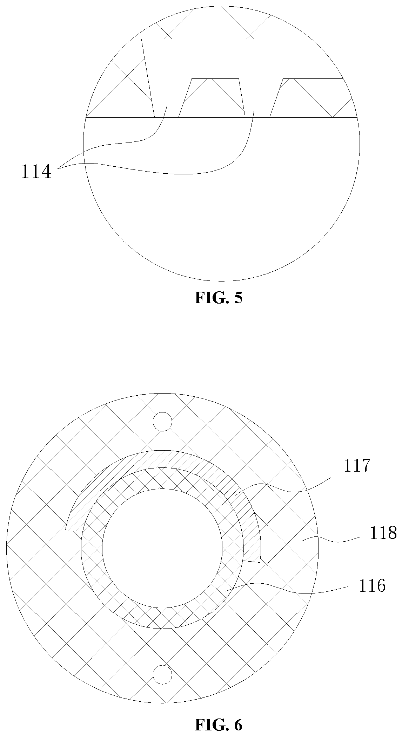

In other embodiments, a plurality of first side holes 114, such as two or three first side holes, are formed on the side wall of the sheath catheter main body 110. In the partial enlarged view of the first side holes as shown in FIG. 5, two first side holes 114 are formed on the sheath catheter main body 110, and the two side holes 114 are arranged along an axial direction of the sheath catheter main body 110 to facilitate the rapid diffusion of the contrast solution or other medicinal liquid along the axial direction of the sheath catheter main body 110.

In other embodiments, a plurality of perfusion lumens and a plurality of first side holes are formed on the side wall of the sheath catheter main body, and each perfusion lumen is in communication with at least one first side hole. The plurality of perfusion lumens are arranged at intervals along the circumferential direction of the sheath catheter main body, and proximal ends of the plurality of perfusion lumens are in communication through a conduit. When the medicine or the contrast solution is inputted from the conduit, the medicine or the contrast solution reaches the plurality of first side holes along the plurality of perfusion lumens. Therefore, the medicine or the contrast solution can rapidly diffuse along the circumferential direction of the sheath catheter main body.

As shown in FIG. 1 and FIG. 6, the sheath catheter main body includes an inner layer 116, a support layer 117 and an outer layer 118 that are arranged radially from inside to outside; and the support layer 117 includes at least one support rod 1171, where the support rod 1171 helically extends along the axial direction of the sheath catheter main body. In other embodiments, the support rod is woven to form the support layer. The inner layer is formed by a PTFE liner, and the outer layer is made of a PEBAX tubing.

The inflation lumen 112 and/or the perfusion lumen 113 are embedded into the outer layer 118. Specifically, the aperture of the inflation lumen 112 or the perfusion lumen 113 is less than the thickness of the outer layer 118. Since the outer layer 118 itself has a particular thickness, embedding the inflation lumen 112 and/or the perfusion lumen 113 into the outer layer 118 can reduce the overall catheter diameter of the sheath catheter main body 110 to facilitate the passage of the balloon sheath catheter 100 within the blood vessel. In other embodiments, the inflation lumen 112 and/or the perfusion lumen 113 are arranged between the inner layer 116 and the support layer 117 or arranged between the support layer 117 and the outer layer 118.

A valve body 130 is provided in the perfusion lumen, and when the fluid pressure in the perfusion lumen 113 is greater than the preset pressure of the valve body 130, the valve body 130 opens to allow fluid to flow from the distal end of the sheath catheter main body 110 to the proximal end of the sheath catheter main body 110. In this embodiment, the preset pressure of the valve body 130 is greater than the pressure of venous blood flow and less than the pressure of arterial blood flow. When the distal end of the balloon sheath catheter 100 is inserted into the blood vessel of the patient, if the blood can pass smoothly through the valve body 130, it indicates that the distal end of the balloon sheath catheter 100 is inserted into the artery of the patient; and if the blood cannot pass through the valve body 130, it indicates that the distal end of the balloon sheath catheter 100 is inserted into the vein of the patient. Therefore, the balloon sheath catheter 100 in this embodiment can assist a surgeon in rapidly determining whether the position where the balloon sheath catheter 100 is inserted is correct.

For ease of observation, the valve body 130 is arranged near the proximal end of the perfusion lumen 113, and when the distal end of the balloon sheath catheter 100 reaches a lesion position, the valve body 130 is located outside the body of the patient. Moreover, in this embodiment, the outer layer 118 is made of transparent PEBAX tubing to enable the surgeon to observe the valve body 130 located in the perfusion lumen 113 through the outer layer. Alternatively, the outer layer 118 is provided with at least one transparent section which is made of transparent PEBAX tubing, and the valve body 130 is arranged inside the transparent section.

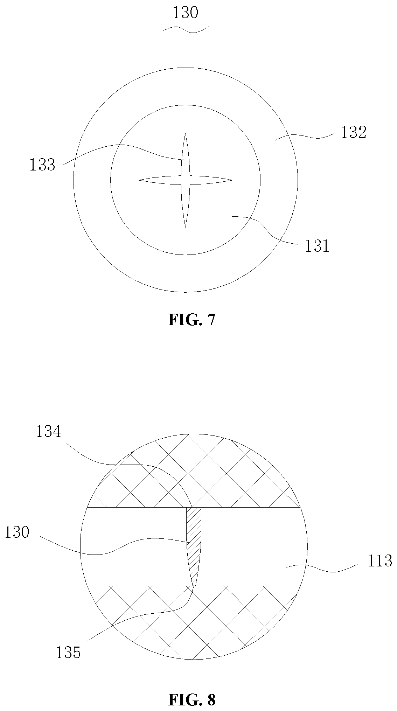

Specifically, as shown in FIG. 7, the valve body 130 in this embodiment is provided with a flow part 131 and a connection part 132. A slit 133 is formed on the flow part 131, the connection part 132 is arranged around the flow part 131, and the connection part 132 is used for connecting the flow part 131 and an inner wall of the perfusion lumen 113. The thickness of the flow part 131 is less than the thickness of the connection part 132. When the flow part 131 is subjected to the pressure of blood and the pressure of the blood is greater than the preset pressure of the valve body 130, the flow part 131 deforms and opens the slit 133, and the blood passes through the valve body 130 from the slit 133.

When the balloon sheath catheter 100 of this embodiment is manufactured, the outer layer 118 can be directly cut so that the valve body 130 can be put into the perfusion lumen 113. After the valve body 130 is fixed to the inner wall of the perfusion lumen 113 by an adhesive or hot melting, a cut on the outer layer is closed by the adhesive or hot melting. By adopting the above mode, the valve body 130 can be accurately put into the perfusion lumen 113, so as to ensure that the valve body 130 is located outside the human body after the balloon sheath catheter 100 is inserted into the human body.

In other embodiments, as shown in FIG. 8, a circumferential edge of the valve body 130 includes a connection section 134 and a movable section 135; the connection section 134 is fixedly connected to an inner wall surface of the perfusion lumen 113; and the movable section 135 can move relative to the inner wall surface of the perfusion lumen 113. When the valve body 130 is subjected to the pressure of the blood and the pressure of the blood is greater than the preset pressure of the valve body 130, the valve body 130 deforms and causes the movable section 135 to move away from the inner wall surface of the perfusion lumen 113, and the blood passes through the valve body 130 through a gap between the movable section 135 and the inner wall surface of the perfusion lumen 113.

### Embodiment 2

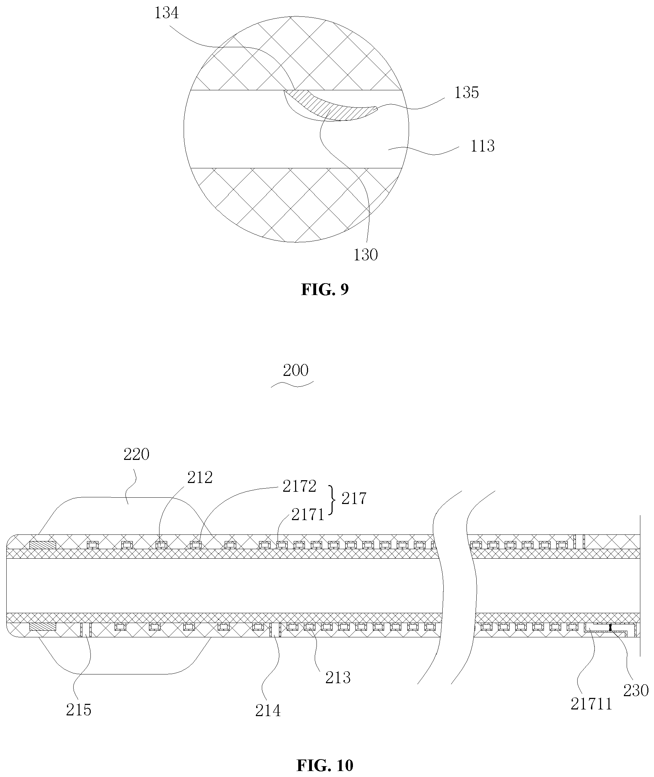

Embodiment 2 differs from the balloon sheath catheter 100 disclosed in embodiment 1 mainly in that, as shown in FIG. 10, a support rod of a balloon sheath catheter 200 in embodiment 2 is a hollow tube, and the lumen of the support rod forms at least one of the perfusion lumen 213 or the inflation lumen 212. The support rod can not only enhance the support strength of the balloon sheath catheter 200, but also provide the perfusion lumen and/or the inflation lumen, thereby reducing the space occupied by the perfusion lumen and/or the inflation lumen on the balloon sheath catheter, which is conducive to further reducing the catheter diameter of the balloon sheath catheter.

In this embodiment, the support layer 217 of the balloon sheath catheter 200 includes a first support rod 2171 and a second support rod 2172; the first support rod 2171 and the second support rod 2172 both extend helically along an axial direction of the balloon sheath catheter 200; and helical rings of the first support rod 2171 are arranged alternately with helical rings of the second support rod 2172. An inner cavity of the first support rod 2171 forms the perfusion lumen 213, and an inner cavity of the second support rod 2172 forms the inflation lumen 212. A distal end of the first support rod 2171 extends to the first side hole 214, and a distal end of the second support rod 2172 extends to the second side hole 215. Therefore, due to having the second support rod 2172 only, the distal end of the balloon sheath catheter 200 has lower strength and better flexibility, thereby avoiding damage to the blood vessels caused by the distal end of the balloon sheath catheter 200.

The first support rod 2171 includes a straight section 21711, the straight section 21711 is arranged on the proximal end of the balloon sheath catheter 200 and extends along the axial direction of the balloon sheath catheter 200, and the valve body 230 is arranged in the straight section 21711. The first support rod 2171 is made of materials such as polyethylene (PE) and nylon, and at least part of the straight section 21711 is made of transparent materials such as PE and nylon. Preferably, the first support rod 2171 is entirely made of transparent materials such as PE and nylon.

### Embodiment 3

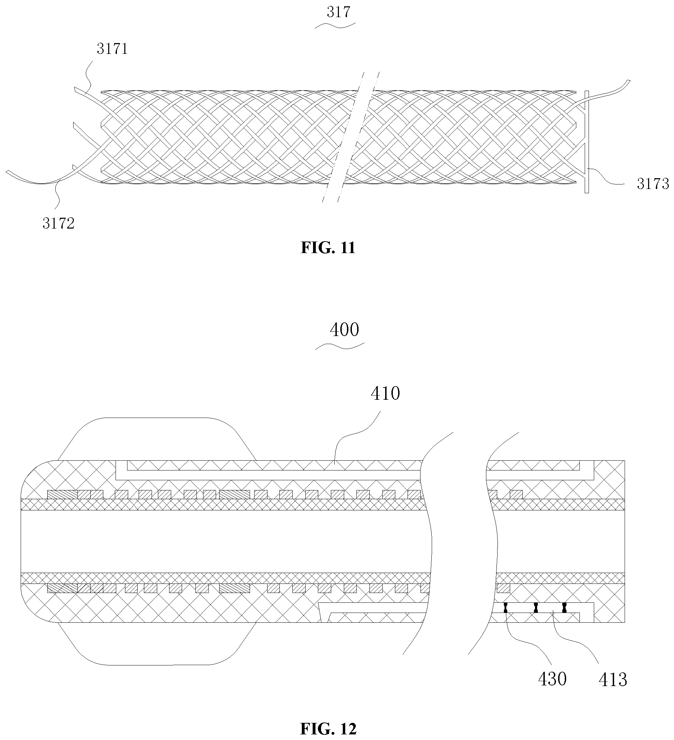

Embodiment 3 differs from the balloon sheath catheter disclosed in embodiment 2 mainly in that, as shown in FIG. 11, the support layer 317 includes at least one first support rod 3171 and at least one second support rod 3172, and the at least one first support rod 3171 and the at least one second support rod 3172 are woven to form the support layer 317. In this embodiment, the support layer 317 includes three first support rods 3171 and one second support rod 3172, and the second support rod 3172 is in communication with the balloon of the balloon sheath catheter for inflating the balloon. The first support rods 3171 are used for injecting the medicine or the contrast agent into the blood vessel. The support layer 317 in this embodiment includes three first support rods 3171, a distal end of each first support rod 3171 corresponds to one first side hole formed on the side wall of the balloon sheath catheter, and the medicine or the contrast solution reaches the three first side holes respectively along the three first support rods 3171; therefore, the medicine or the contrast solution can rapidly diffuse along the circumferential direction of the sheath catheter main body.

Proximal ends of the three first support rods 3171 are in communication through an annular sector conduit 3173, the conduit 3173 is in communication with a second angiographic joint of the balloon sheath catheter, and a surgeon injects the contrast solution or the medicine through the second angiographic joint.

### Embodiment 4

Embodiment 4 differs from the balloon sheath catheter 100 disclosed in embodiment 1 mainly in that, as shown in FIG. 12, a plurality of valve bodies 430 are provided in a perfusion lumen 413 of a balloon sheath catheter 400 in embodiment 4, and the preset pressures of the plurality of valve bodies 430 increase gradually along a direction from a distal end to a proximal end of a sheath catheter main body 410.

The plurality of valve bodies 430 are all arranged on a proximal end of the balloon sheath catheter 400, and in the process of a surgery, the plurality of valve bodies 430 are located outside the body of the patient and are visible through the balloon sheath catheter 400. When the balloon sheath catheter 400 is inserted into a blood vessel, the current blood pressure of the patient can be monitored in real time by the valve body 430 to which the blood flows back, thereby preventing dangerous situations from happening (for example, the blood pressure of the patient suddenly increases, which may be stress-induced elevation of blood pressure, may easily lead to various complications such as cerebral hemorrhage and heart failure, and needs timely intervention to lower the blood pressure; and the blood pressure of the patient suddenly decreases, which may be due to vascular rupture or excessive loss of blood, may easily lead to various complications such as insufficient circulating blood volume and multiple organ dysfunction syndrome, and needs timely intervention to raise the blood pressure).

If angiography or thrombolysis is required during the surgery, the contrast solution or the medicinal liquid can be injected into the perfusion lumen 413 by a pressure greater than the current blood pressure. At this time, blood pressure monitoring is temporarily suspended. After the injection is stopped, the blood flow flows back to the perfusion lumen, and the function of blood pressure monitoring is restored.

In this embodiment, the balloon sheath catheter 400 includes three valve bodies 430, the preset pressures of the three valve bodies 430 are 20 mmHg, 40 mmHg, and 60 mmHg respectively, and the three valve bodies 430 are arranged at intervals from small to large along the distal end to the proximal end of the balloon sheath catheter 400 according to the sizes of the preset pressures. The valve bodies 430 are made of rubber or silicone, and the preset pressures of the valve bodies are in direct proportion to the thicknesses thereof. For example, the thickness of the valve body with the preset pressure of 40 mmHg is twice that of the valve body with the preset pressure of 20 mmHg.

The technical features of the above embodiments can be arbitrarily combined. To make the description concise, all possible combinations of the technical features in the above embodiments are not described. However, as long as the combinations of the technical features do not have contradictions, the combinations shall be considered to be the scope disclosed in this description.

The above embodiments only express several implementation modes of the present invention, and are described more specifically in details, but shall not be consequently interpreted as a limitation to the scope of the patent for invention. It should be noted that, for those ordinary skilled in the art, several deformations and improvements can also be made without departing from the concept of the present invention, all of which belong to the protection scope of the present invention. Therefore, the protection scope of the patent for the present invention shall be subject to appended claims.

## Claims

1. A balloon sheath catheter, comprising:
a sheath catheter main body, which is provided with a main lumen, an inflation lumen and a perfusion lumen that are not in communication with each other, wherein a distal end of the main lumen penetrates through a distal end of the sheath catheter main body, at least one first side hole and at least one second side hole are formed on a side wall of the sheath catheter main body, and the first side hole is in communication with the perfusion lumen and an external space of the sheath catheter main body; and
a balloon, which is arranged near a distal end of the balloon sheath catheter, wherein the second side hole is in communication with the balloon and the inflation lumen, and the first side hole is formed on a proximal side of the balloon.

2. The balloon sheath catheter according to claim 1, wherein
the aperture of the first side hole gradually decreases from inside to outside along a radial direction of the sheath catheter main body.

3. The balloon sheath catheter according to claim 1, wherein
the sheath catheter main body comprises an inner layer, a support layer and an outer layer that are arranged radially from inside to outside;
the support layer comprises at least one support rod, wherein the support rod helically extends along an axial direction of the sheath catheter main body, or the support rod is woven to form the support layer.

4. The balloon sheath catheter according to claim 3, wherein
the inflation lumen and/or the perfusion lumen are embedded into the outer layer.

5. The balloon sheath catheter according to claim 3, wherein
the support rod is a hollow tube, and the lumen of the support rod forms at least one of the perfusion lumen or the inflation lumen.

6. The balloon sheath catheter according to claim 3, wherein
a valve body is provided in the perfusion lumen, and when the fluid pressure in the perfusion lumen is greater than the preset pressure of the valve body, the valve body opens to allow fluid to flow from the distal end of the sheath catheter main body to a proximal end of the sheath catheter main body.

7. The balloon sheath catheter according to claim 6, wherein
a plurality of valve bodies are provided in the perfusion lumen, and the preset pressures of the plurality of valve bodies increase gradually along a direction from the distal end to the proximal end of the sheath catheter main body.

8. The balloon sheath catheter according to claim 6, wherein
the valve body is arranged near a proximal end of the perfusion lumen.

9. The balloon sheath catheter according to claim 6, wherein
the outer layer comprises at least one transparent section, and the projection of the valve body along the radial direction of the sheath catheter main body falls on the transparent section to facilitate the observation of the valve body through the transparent section.

10. The balloon sheath catheter according to claim 1, wherein
the balloon sheath catheter further comprises a first angiographic joint and a second angiographic joint; the first angiographic joint is arranged on a proximal side of the sheath catheter main body and is in communication with the main lumen; and the second angiographic joint is arranged on the proximal side of the sheath catheter main body and is in communication with the perfusion lumen.
